Europäisches Patentamt

European Patent Office

Office européen des brevets

(11) Veröffentlichungsnummer: **0 058 426**
**B1**

(12) **EUROPÄISCHE PATENTSCHRIFT**

(45) Veröffentlichungstag der Patentschrift:
21.12.83

(21) Anmeldenummer: 82101132.7

(22) Anmeldetag: 16.02.82

(51) Int. Cl.³: **C 12 P 7/06, C 12 N 1/16**

(54) Verfahren zur Herstellung von Äthanol durch Fermentation von Kohlenhydraten.

(30) Priorität: 16.02.81 DE 3105581

(43) Veröffentlichungstag der Anmeldung:
25.08.82 Patentblatt 82/34

(45) Bekanntmachung des Hinweises auf die Patenterteilung:
21.12.83 Patentblatt 83/51

(84) Benannte Vertragsstaaten:
AT BE CH DE FR GB IT LI LU NL SE

(56) Entgegenhaltungen:
DE - B - 2 845 387
US - A - 4 032 407
US - A - 4 046 921

CHEMICAL ABSTRACTS, Band 94, Nr. 13, Juni 1981,
Seite 466, Nr. 207160p, Columbus, Ohio, USA

(73) Patentinhaber: Moebus, Otto, Dr., Lämmerstücken 36,
D-2300 Kiel-Russee (DE)
Patentinhaber: Teuber, Michael, Prof. Dr.,
Gartenstrasse 114, D-2302 Flintbek (DE)
Patentinhaber: Reuter, Helmut, Prof. Dr. Ing.,
Dorfstede 23, D-2300 Kiel-Schulensee (DE)

(72) Erfinder: Moebus, Otto, Dr., Lämmerstücken 36,
D-2300 Kiel-Russee (DE)
Erfinder: Teuber, Michael, Prof. Dr., Gartenstrasse 114,
D-2302 Flintbek (DE)
Erfinder: Reuter, Helmut, Prof. Dr. Ing., Dorfstede 23,
D-2300 Kiel-Schulensee (DE)

(74) Vertreter: Ruschke, Hans Edvard et al, c/o Ruschke &
Partner Patentanwälte Plenzenauerstrasse 2,
D-8000 Munchen 80 (DE)

## Verfahren zur Herstellung von Äthanol durch Fermentation von Kohlenhydraten

Der biotechnologische Prozeß zur Herstellung von Äthanol wird in den Spiritusfabriken so durchgeführt, daß eine Maische aus aufgeschlossenem kohlenhydrathaltigem Material vergoren und anschließend in einer Maischesäule durch Heizung mit direktem Dampf vollkommen vom Alkohol befreit wird und daß das entweichende Wasser-Alkohol-Gemisch einer Rektifikationssäule zugeführt wird. Als Restlösung fließt aus der Maischesäule eine Schlempe ab, deren Verwertung ein Problem darstellt. Nach einer im Auftrag des Bundesministeriums für Forschung und Technologie ausgearbeiteten DECHEMA-Studie über Forschung und Entwicklung in der Biotechnologie S. 121—122 (1974) fallen in der Bundesrepublik jährlich ca. 2 Millionen $m^3$ Schlempen aus stärkehaltigen Rohstoffen an, die mit 25 000 mg $O_2$/l einen sehr hohen $BSB_5$-Wert (Biochemischer Sauerstoffbedarf in 5 Tagen) aufweisen. Die Schlempen sind nicht lagerfähig und können deshalb nur direkt verfüttert werden. Eine Trocknung des Produktes zum Zwecke der Zumischung in Futtermittel wäre erwünscht. Nach K. R. Dietrich (siehe H. Kretzschmar, Hefe und Alkohol, Springer-Verlag, Berlin, Göttingen, Heidelberg, S. 506 bis 509, 1955) kann die am Fuße der Maischesäule ablaufende Schlempe über ein Sieb geführt, die abgesiebten Feststoffe abgepreßt, die anfallende Flüssigkeit im Mehrstufenverdampfer eingedampft und die eingeengte Masse mit Walzentrocknern getrocknet werden. Nach der oben zitierten DECHEMA-Studie ist in der Bundesrepublik eine derartige Trocknung der Schlempe aus energiewirtschaftlichen Gründen bei den vorherrschenden Betriebsgrößen unwirtschaftlich.

Eine der der Erfindung zugrunde liegenden Aufgaben ist die Produktion von Äthanol unter solchen Verfahrensbedingungen, daß keine wasserreiche Schlempe anfällt.

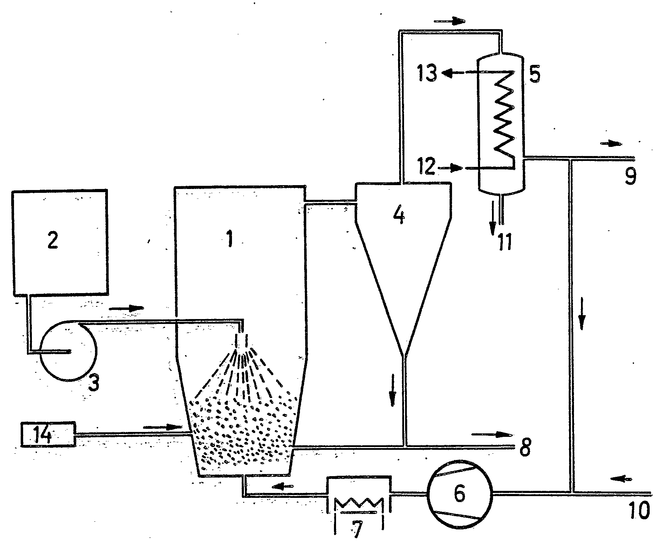

Diese Aufgabe wird durch das in Anspruch 1 angegebene Verfahren gelöst, dessen bevorzugte Ausgestaltungen den Gegenstand der Unteransprüche 2 bis 11, der Beispiele und der Figur bilden.

Die Äthanolproduktion wird erfindungsgemäß in einem mit Gas fluidisierten Bett (Fließbett, Wirbelschicht) durchgeführt, dessen Partikelfraktion aus feuchter Mikroorganismenmasse, z. B. Saccharomyces cerevisiae, besteht. Auf die fluidisierten Partikel wird vorzugsweise eine Nährlösung mit fermentierbaren Kohlenhydraten aufgesprüht.

Das mit dem Wasser abgedampfte Äthanol wird in einem nachgeschalteten Kondensator abgeschieden, so daß das fluidisierte Bett sowohl als Bioreaktor als auch zusammen mit dem Kühlsystem als einstufige Destillationsanlage arbeitet. Die bei Submersverfahren zur Spiritusherstellung verwendete Maischesäule entfällt somit im erfindungsgemäßen Verfahren.

Anstelle der Schlempe wird ein proteinreiches Produkt mit 30—40% Trockenmasse gebildet, das bei kontinuierlicher Arbeitsweise in zeitlichen Abständen dem Bioreaktor entnommen und bis zur gewünschten Restfeuchte weiter getrocknet werden kann.

Die Vermehrung der Mikroorganismen wird über den Sauerstoffgehalt der Gasphase, die Äthanolbildung auch über den Zulauf der Nährlösung eingestellt. Das zur Fluidisierung verwendete Gas wird vorzugsweise in das Bett rezirkuliert. Der Sauerstoffpartialdruck kann durch Zumischen anderer Gase zu Luft, z. B. von Stickstoff oder von Kohlendioxid, insbesondere von Kohlendioxid, das die Hefe selbst bildet, reguliert werden.

Die mit der Erfindung erzielbaren Vorteile bestehen insbesondere darin, daß bei Durchführung der Äthanolproduktion in einem durch Gas fluidisierten Bett mit anschließender Kondensation des Wasser-Alkohol-Gemisches die bisher notwendige Maischesäule entfällt. Das mit Gas fluidisierte Bett übernimmt in Verbindung mit dem Kühlsystem die Funktion der ersten Destillationsstufe. Eine wasserreiche Schlempe fällt nicht an, die überschüssige Hefe kann zusammen mit dem restlichen nicht fermentierten Substrat in Form eines Granulates aus dem fluidisierten Bett entnommen werden. Das Granulat enthält nur so viel Wasser, wie zur Aufrechterhaltung der Enzymfunktionen der Mikroorganismen (z. B. Hefen) erforderlich ist. In einem sich anschließenden Trocknungsvorgang — beim Chargenbetrieb z. B. in der gleichen Anlage, bei kontinuierlichem Betrieb in einer zweiten Stufe — kann das Granulat bis zur gewünschten Restfeuchte getrocknet werden.

Es kann u. U. von wirtschaftlichem Vorteil sein, daß das vorgeschlagene Verfahren als kombiniertes Verfahren zur Produktion von Äthanol bei gleichzeitiger Herstellung von Einzellerprotein eingerichtet wird. Bei Verwendung von Hefen (Saccharomyces cerevisiae) kann die Menge des mit dem Granulat anfallenden Einzellerproteins durch Erhöhung des Sauerstoffpartialdruckes in dem für die Fluidisierung verwendeten Gas auf Kosten der Äthanolausbeute erhöht werden (Pasteur-Effekt). Eine Verminderung der Ausbeute an Einzellerprotein zugunsten einer Äthanolbildung kann auch bei erhöhtem Sauerstoffpartialdruck im fluidisierten Bett durch Zufuhr eines Überschusses an Zucker (Glucose) erreicht werden (Crabtree-Effekt).

Beim konventionellen Chargenbetrieb zur Spiritusfabrikation nimmt die Fermentationsrate mit steigender Alkoholkonzentration in der Maische immer mehr ab, da Alkohol in höheren Konzentrationen die Enzymwirkung der Hefe hemmt. In dem hier dargestellten System wird der gebildete Alkohol kontinuierlich im Gasstrom von der Oberfläche der Partikel abgedampft, wodurch die Alkoholkonzentration in den Partikeln niedrig bleibt und die Fermentation mit maximaler Geschwindigkeit abläuft. Nach Kretzschmar (Technische Mikrobiologie, Paul Parey, Berlin, Hamburg. S. 36, 1968) ist unter den Bedingungen einer technischen Vergärung die Alkoholkonzentration im abgedampften Wasser (d. h. die Konzentration an Äthanol im Kondensat) gegenüber der Konzentration im Fermenter um etwa das Zehnfache angereichert.

Die beim konventionellen Verfahren der Spiritusherstellung notwendige Kühlung des Fermentationstanks entfällt bei der Produktion im fluidisierten Bett, da die entwickelte Wärme direkt für die Wasserverdampfung ausgenützt wird (siehe O. Moebus, M. Teuber und H. Reuter, DE-PS 2 845 387).

Mit einem fluidisierten Bett könnte möglicherweise auch eine Äthanolherstellung zwecks Verwendung als Kraftstoff auf der Basis von Zuckerrüben durchgeführt werden, wie es von K. D. Kirby und C. J. Mardon vorgeschlagen wird (Biotechnology and Bioengineering 12, S. 2425 – 2427, 1980).

Die Erfindung wird nachfolgend anhand eines Ausführungsbeispiels beschrieben. Die Figur zeigt schematisch eine Anlage, vorgesehen für die kombinierte Produktion von Äthanol und Einzellerprotein im fluidisierten Bett (1), das zu Beginn mit den rieselfähigen Mikroorganismen-Partikeln aus (14) beschickt und dem aus dem Vorratsbehälter für eine kohlenhydrathaltige wäßrige Nährlösung (2) mit einer Pumpe (3) einer Sprühdüse, angebracht über dem fluidisierten Bett, Substrat zugeführt wird. Mit dem Zyklon (4) werden aus dem Bett ausgetragene Feststoffanteile abgeschieden. In den Oberteil des Zyklons ist eine $O_2$-Elektrode zur Messung des $O_2$-Partialdruckes im Gas eingebaut (nicht gezeichnet). Die aus dem fluidisierten Bett entnommenen Einzellerproteinpartikel und die Feststoffanteile aus dem Zyklon können zu einer Fraktion zusammengefaßt werden (8). In einem Kühler (5) wird das Wasser-Alkohol-Gemisch kondensiert und bei (11) entnommen. Die Kühlung des Gases kann mit Brunnenwasser oder mit Eiswasser erfolgen, Eintritt des Wassers bei (12), Austritt bei (13). Das aus dem fluidisierten Bett rezirkulierte Gas bzw. frisches Gas (10) wird mit einem Ventilator (6) angesaugt, über ein Heizsystem (7) erwärmt und dann in das Bett geleitet. Bei (9) kann gekühltes Gas entweichen.

Die über die Pumpe (3) zugeführte Nährlösung enthielt für die beiden nachfolgend beschriebenen technischen Versuche 250 g Glucose (Maizena, Handelsprodukt, mit einem Zusatz von Vitaminen), 10 g Hefeextrakt, 4,5 g $KH_2PO_4$, 1,0 g $MgHPO_4 \cdot 3 H_2O$ und 0,2 g $CaCl_2 \cdot 2 H_2O$. Die Bedingungen im fluidisierten Bett wurden bei beiden Versuchen wie folgt eingestellt: Gasgeschwindigkeit im Bett 0,54 m/s, Temperatur des Gases vor Eintritt in das Bett 45°C, relative Feuchte des Gases vor Eintritt in das Bett 10%, Temperatur des Gases nach Austritt aus dem Bett 19°C. Die Kühlwassertemperatur vor Eintritt in den Kühler betrug 1°C, nach Austritt 6°C, bei einer Kühlwassermenge von 450 l/h. Durch Einleiten von Stickstoff in die Anlage wurde der $O_2$-Gehalt des Gases beim ersten Versuch auf 7% eingestellt, beim zweiten Versuch betrug der $O_2$-Gehalt des rezirkulierten Gases 0%. Die eingesetzte Hefemenge, mit einer Küchenmaschine gereibselte Backhefe der Fa. UNIFERM, Monheim, der zur Verbesserung der Rieselfähigkeit der Partikel 0,8% Cab-o-sil der Fa. Cabot (pyrogene Kieselsäure) zugesetzt wurden, betrug beim ersten Versuch 2,930 kg, beim zweiten Versuch 2,817 kg. Die Auswertung ergab:

1. Bilanz des ersten Versuches (7% $O_2$ im Gas). Pro l Nährlösung, in der durch enzymatische Analyse 226,6 g Glucose/l bestimmt wurde, bildeten sich 29,8 g Äthanol und 48,3 g Hefetrockenmasse (HTS). Die Äthanolproduktion betrug 10,7 g Äthanol/kg Partikel und Stunde und, bei einem spezifischen Gewicht der Partikel von 0,9, ergeben sich 11,9 g Äthanol/l Partikel und Stunde. Der theoretische Glucosebedarf für die Äthanolbildung (bei Verwendung eines Faktors von 1,956) betrug 58,3 g Glucose/l Nährlösung, für die HTS-Bildung (bei Verwendung eines unter voll aeroben Bedingungen erhaltenen Ausbeutekoeffizienten von 0,456 g HTS/100 g Glucose) 105,9 g Glucose/l Nährlösung und für die Restglucose in den Partikeln ergab sich nach Umrechnung pro l Nährlösung 0,4 g Glucose/l. Dementsprechend ergab sich, bezogen auf die eingesetzte Glucose, eine technische Ausbeute von 73%. Die Versuchsdauer betrug 4,73 h.

2. Bilanz des zweiten Versuches (0% $O_2$ im Gas). Pro l Nährlösung mit 237,0 g Glucose/l (Bestimmung enzymatisch), wurden 52,9 g Äthanol und 35,3 g HTS gebildet. Die Äthanolproduktion betrug 20,7 g Äthanol/kg Partikel und Stunde, bzw. 23,0 g Äthanol/l Partikel und Stunde. Der theoretische Glucosebedarf für die Äthanolbildung betrug 103,5 g/l Nährlösung, für die HTS-Bildung 77,4 g Glucose/l Nährlösung und für die Restglucose in den Partikeln ein Wert von 9,0 g Glucose, umgerechnet auf 1 l Nährlösung. Bezogen auf die eingesetzte Glucose, ergibt sich eine technische Ausbeute von 80%. Die Versuchsdauer betrug 5,65 h.

M. Moo-Young, J. Lamptey und C. W. Robinson (Biotechnology Letters, 12, S. 541 – 548, 1980) finden eine Äthanolproduktivität von 21,8 g/l · h unter Verwendung von trägergebundener Saccharomyces cerevisiae, die im Bereich des Wertes unseres zweiten Versuches liegt und in gerührten Submers-Systemen unter optimalen Bedingungen dann gefunden wird, wenn die Zellmasse in den Fermenter zurückgeführt wird (18 – 32 g/l · h).

Die Äthanolanalyse im Kondensat (2. Versuch) ergab nach der enzymatischen Methode (Äthanol-UV-Test, Boehringer, Mannheim) 48,4 g Äthanol/l Kondensat, nach der kolorimetrischen Methode durch Oxidation mit Bichromat in schwefelsaurer Lösung (B. Lange, Kolorimetrische Analyse, Verlag Chemie, Weinheim-Bergstr., S. 274 – 275, 1952) 48,0 g Äthanol/l Kondensat. Da nach der ersten Methode spezifisch das Äthanol erfaßt wird, nach der zweiten Methode allgemein die abgedampften oxidablen Substanzen gemessen werden, darf man annehmen, daß unter den gewählten Fermentationsbedingungen Äthanol als Hauptprodukt neben der Biomasse gebildet wird, ohne daß andere Gärprodukte mengenmäßig in die Bilanz eingehen.

## Patentansprüche

1. Verfahren zur Herstellung von Äthanol und gegebenenfalls Einzellerprotein durch Fermentation von Kohlenhydraten, dadurch gekennzeichnet, daß zur Äthanolproduktion geeignete Mikroorganismen in Form riesalfähiger Partikel in ein mit Gas fluidisiertes Bett gegeben und eine oder mehrere fermentierbare Kohlenhydrate enthaltende wäßrige Nährlösungen in das Bett der fluidisierten Partikel geleitet und darin fermentiert werden, während die Gärungsbedingungen im wesentlichen durch die Temperatur und den Sauerstoffpartialdruck des als kontinuierliche Phase in das Wirbelbett strömenden Gases eingestellt werden, und daß das Äthanol/Wasser-Gemisch über Bausteine der Kühl- und Fraktioniertechnik aus dem den fluidisierten Partikeln entströmenden Gas abgeschieden und aufbereitet wird.

2. Verfahren nach Anspruch 1, dadurch gekennzeichnet, daß das in das fluidisierte Bett einströmende Gas vor seinem Eintritt über ein Heizelement erwärmt wird.

3. Verfahren nach Anspruch 1 oder 2, dadurch gekennzeichnet, daß die nichtfermentierte Biomasse, zusammen mit dem restlichen nichtfermentierten Substrat, in Form von noch feuchtem Granulat aus dem fluidisierten Bett entnommen und das Granulat in einem Trocknungsgang bis zur gewünschten Restfeuchte getrocknet wird.

4. Verfahren nach einem der Ansprüche 1 bis 3, dadurch gekennzeichnet, daß Saccharomyces-Rassen als Mikroorganismen eingesetzt werden.

5. Verfahren nach einem der Ansprüche 1 bis 4, dadurch gekennzeichnet, daß das Verhältnis der produzierten Äthanolmengen zur Synthese von Einzellerprotein durch Regelung des Sauerstoffpartialdruckes im Gas der kontinuierlichen Phase eingestellt wird.

6. Verfahren nach einem der Ansprüche 1 bis 5, dadurch gekennzeichnet, daß als Substrate Nährlösungen aus Rohstoffen, die hydrolysierte Stärke und/oder hydrolysierte Cellulose enthalten, verwendet werden.

7. Verfahren nach Anspruch 5, dadurch gekennzeichnet, daß zur Einstellung des Sauerstoffpartialdruckes im fluidisierten Bett Stickstoff und/oder zusätzliches Kohlendioxid in das Bett eingeleitet werden.

8. Verfahren nach Anspruch 5, dadurch gekennzeichnet, daß in das fluidisierte Bett zusätzlich Luft eingeleitet wird, deren Sauerstoff durch die Mikroorganismen durch Veratmung eines Teiles der Kohlenhydratquelle teilweise oder vollständig verbraucht wird, und daß das aufgearbeitete Gas in das fluidisierte Bett rezirkuliert wird.

9. Verfahren nach einem der Ansprüche 1 bis 8, dadurch gekennzeichnet, daß die Menge des pro Zeiteinheit im fluidisierten Bett verdampften Wassers bzw. der Wassergehalt der Partikel im fluidisierten Bett über den Taupunkt des Wasserdampf-Gas-Gemisches im Kühlsystem für zu rezirkulierendes Gas eingestellt wird.

10. Verfahren nach einem der Ansprüche 1 bis 9, dadurch gekennzeichnet, daß das durch einen Kühler abgeschiedene Kondensat aus Wasser und Äthanol einer nachgeschalteten Rektifikationskolonne zugeführt wird.

11. Verfahren nach einem der Ansprüche 1 bis 10, dadurch gekennzeichnet, daß die Menge des pro Zeiteinheit im fluidisierten Bett verdampften Wassers über das Heizelement und die an das Gas abgegebene Wärmemenge geregelt wird.

## Claims

1. Method of producing ethanol and, given the case, single cell protein by the fermentation of carbohydrates, characterized by adding microorganisms suited for the production of ethanol in the form of flowable particles to a gas-fluidized bed and by passing into and fermenting in said bed of fluidized particles aqueous nutrient solutions containing one or more fermentable carbohydrates, by conducting the fermentation while adjusting the fermentation conditions substantially by setting the temperature and the oxygen partial pressure of the gas streaming into the fluidized bed as a continuous phase, and by separating and processing the ethanol-water mixture from the gas flowing from the fluidized particles by means of refrigerating and fractionating systems components.

2. Method as in claim 1, characterized by heating the gas flowing into the fluidized bed by means of a heating element prior to the entry thereof into said bed.

3. Method as in claim 1 or 2, characterized by withdrawing the non-fermented biomass from the fluidized bed in the form of a still moist granulate together with the remainder of the non-fermented substrate, and by drying the granulate to the desired residual moisture content in one drying stage.

4. Method as in any one of claims 1 to 3, characterized by using as microorganisms races of saccharomyces.

5. Method as in any one of claims 1 to 4, characterized by adjusting the ratio of the ethanol production to the synthesis of single cell protein by regulating the oxygen partial pressure in the continuous gas phase.

6. Method as in any one of claims 1 to 5, characterized by using as substrate nutrient solutions of raw materials containing hydrolyzed starch and/or hydrolyzed cellulose.

7. Method as in claim 5, characterized by passing nitrogen and/or additional carbon dioxide into the fluidized bed for adjusting the oxygen partial pressure therein.

8. Method as in claim 5, characterized by passing into the fluidized bed additional air of which the oxygen is consumed partly or completely by said microorganisms in the respiration of part of the carbon hydrate source, and by recirculating the processed gas to the fluidized bed.

9. Method as in any one of claims 1 to 8, characterized by adjusting the amount of water evaporated per unit time in the fluidized bed or the water content of the particles in the fluidized bed via the dew point of the steam — gas mixture in the recirculating gas refrigerating system.

10. Method as in any one of claims 1 to 9, characterized by feeding the cooler-separated condensate consisting of water and ethanol to a subsequent rectifying column.

11. Method as in any one of claims 1 to 10, characterized by regulating the amount of water evaporated per unit time in the fluidized bed via the heating element and the amount of heat passed on to the gas.

## Revendications

1. Procédé de préparation d'éthanol et éventuellement de protéine d'organisme unicellulaire par fermentation d'hydrates de carbone, caractérisé en ce que, pour la production d'éthanol, des microorganismes appropriés sont versés sous forme de particules capables de s'écouler dans un lit fluidisé par du gaz, en ce que des solutions nutritives aqueuses, contenant un ou plusieurs hydrates de carbone fermentables, sont amenés dans le lit des particules fluidisées et fermentées dans ce dernier, tandis que les conditions de fermentation sont ajustées essentiellement par la température et la pression partielle d'oxygène du gaz qui s'écoule dans le lit fluidisé sous la forme d'une phase continue, et en ce que le mélange d'éthanol/eau est séparé à partir du gaz, qui se dégage des particules fluidisées, et est traité par l'intermédiaire d'éléments modulaires de la technique de refroidissement et de fractionnement.

2. Procédé suivant la revendication 1, caractérisé en ce que le gaz pénétrant dans le lit fluidisé est chauffé avant son entrée par l'intermédiaire d'un élément chauffant.

3. Procédé suivant l'une des revendications 1 et 2, caractérisé en ce que la biomasse non fermentée est, conjointement avec le substrat non fermenté résiduaire, prélevée du lit fluidisé sous la forme d'un produit de granulation encore humide et en ce que le produit de granulation est séché jusqu'au taux d'humidité résiduaire souhaité, en une opération de séchage.

4. Procédé suivant l'une des revendications 1 à 3, caractérisé en ce que des espèces de Saccharomyces sont mises en oeuvre comme microorganismes.

5. Procédé suivant l'une des revendications 1 à 4, caractérisé en ce que le rapport entre les quantités produites d'éthanol et la quantité synthétisée de protéine d'organisme unicellulaire est ajusté par réglage de la pression partielle d'oxygène dans le gaz de la phase continue.

6. Procédé suivant l'une des revendications 1 à 5, caractérisé en ce qu'on utilise, comme substrat, des solutions nutritives à base de matières premières qui contiennent de l'amidon hydrolysé et/ou de la cellulose hydrolysée.

7. Procédé suivant la revendication 5, caractérisé en ce que de l'azote et/ou du dioxyde de carbone supplémentaire sont introduits dans le lit pour ajuster la pression partielle d'oxygène dans le lit fluidisé.

8. Procédé suivant la revendication 5, caractérisé en ce que, dans le lit fluidisé, on introduit de l'air supplémentaire dont l'oxygène est consommé partiellement ou totalement par les microorganismes par respiration d'une partie de la source d'hydrate de carbone et en ce que le gaz traité est recyclé dans le lit fluidisé.

9. Procédé suivant l'une des revendications 1 à 8, caractérisé en ce que la quantité de l'eau évaporée par unité de temps dans le lit fluidisé ou respectivement la teneur en eau des particules dans le lit fluidisé est ajustée au-dessus du point de condensation du mélange vapeur d'eau-gaz dans le système de refroidissement du gaz à recycler.

10. Procédé suivant l'une des revendications 1 à 9, caractérisé en ce qu'un produit de condensation séparé par un dispositif de refroidissement et à base d'eau et d'éthanol est amené à une colonne de rectification montée en aval.

11. Procédé suivant l'une des revendications 1 à 10, caractérisé en ce que la quantité de l'eau évaporée par unité de temps dans le lit fluidisé est réglée par l'intermédiaire de l'élément chauffant et par la quantité de chaleur cédée au gaz.